# EUROPEAN PATENT APPLICATION

(11) **EP 1 024 193 A1**
(43) Date of publication of application: **02.08.2000**
(21) Application number: 98933895.9
(22) Date of filing: 22.07.1998
(51) Int. Cl.: C12N 15/60, C12N 15/63, C12P 21/08, C12Q 1/68

(54) **METHOD FOR GENE SCREENING WITH THE USE OF NUCLEAR RECEPTORS**

(30) Priority: 22.07.1997 JP 21262497
(71) Applicant: CHUGAI SEIYAKU KABUSHIKI KAISHA, Tokyo, 115-8543 (JP)
(72) Inventor: KATO, Shigeaki, Saitama 359-1121 (JP); TAKEYAMA, Ken-ichi, Tokyo 114-0003 (JP); KITANAKA, Sachiko, Suginami-ku, Tokyo 166-0003 (JP)
(74) Representative: VOSSIUS & PARTNER
(86) International application number: JP9803280
(87) International publication number: WO9905292

(57) **Abstract**

A system in which a ligand is formed by the expression of a polypeptide that converts a ligand precursor into a ligand, and the ligand thus formed binds to a nuclear receptor to thereby induce the expression of a reporter gene located downstream of the target sequence is constructed. Searching a gene library using this system can isolate a gene encoding a polypeptide capable of converting a ligand precursor into a ligand. This system, which takes the advantage of the transcriptional regulatory function of a nuclear receptor, enables screening a ligand that binds to a nuclear receptor and to examine whether or not a test compound is a ligand that binds to the nuclear receptor, and also screening genes that encode polypeptides capable of converting an inactive form of a wide range of transcriptional regulatory factors into an active form.

## Description

### Technical Field

This invention relates to a method for screening a compound using the nature of transcriptional regulatory factors, mainly nuclear receptors, and a method for determining the compound. Specifically, it relates to a method for screening a gene encoding a polypeptide that converts a ligand precursor into a ligand, a polypeptide that converts a ligand precursor obtainable by the screening method into a ligand, a gene encoding the polypeptide, and a method for determining whether or not a test gene encodes a polypeptide that converts a ligand precursor into a ligand. In addition, it relates to a method for screening a ligand that binds to a nuclear receptor, a ligand obtainable by the screening method, and a method for determining whether or not a test compound is a ligand that binds to a nuclear receptor. Furthermore, it relates to a method for screening a gene encoding a polypeptide that converts an inactive form of a transcriptional regulatory factor into an active form.

### Background Art

1α,25-Dihydroxyvitamin D₃ (1α,25(OH)₂D₃)(A. W. Norman, J. Roth, L. Orchi, Endocr. Rev. 3, 331 (1982); H. F. DeLuca, Adv. Exp. Med. Biol. 196, 361 (1986); M. R. Walters, Endocr. Rev. 13, 719 (1992)) is a hormone form of vitamin D and the most biologically active natural metabolite. This compound is generated by sequential hydroxylation. First, it is hydroxylated in the liver to generate 25-hydroxyvitamin D₃ (25(OH)D₃), then subsequently hydroxylated in the kidney to generate 1α,25(OH)₂D₃ (H. Kawashima, S. Torikai, K. Kurokawa, Proc. Natl. Acad. Sci. USA 78, 1199 (1981); H. L. Henry et al., J. Cell. Biochem. 49, 4 (1992)). The transactivation effect of vitamin D receptor (VDR) is provoked by the binding of 1α,25(OH)₂D₃ to VDR (M. Beato, P. Herrlich, G. Schuts, Cell 83, 851 (1995); H. Darwish and H. F. DeLuca, Eukariotic Gene Exp. 3, 89 (1993); B. D. Lemon, J. D. Fondell, L. P. Freedman, Mol. Cell. Biol. 17, 1923 (1997)). This regulates the transcription of a series of target genes involved in the major functions of vitamin D, such as calcium homeostasis, cell differenciation, and cell proliferation (D. D. Bikle and S. Pillai, Endoc. Rev. 14, 3 (1992); R. Bouillon, W. H. Okamura, A. W. Norman, Endoc. Rev. 16, 200 (1995); M. T. Haussler et al., Recent Prog. Horm. Res. 44, 263 (1988); P. J. Malloy et al., J. Clin. Invest. 86, 2071 (1990)). The importance of the hydroxylation of 25(OH)D₃ in the kidney in the synthesis of active vitamin D has been shown, and it has been believed for a long time that the hydroxylation is done by 25(OH)D₃-1α hydroxylase (1α(OH)-ase), which is localized especially at proximal renal tubules. The activity of 1α(OH)-ase is negatively regulated by its final product, 1α,25(OH)₂D₃ (Y. Tanaka and H. F. DeLuca, Science 183, 1198 (1974); K. Ikeda, T. Shinki, A. Yamaguchi, H. F. DeLuca, K. Kurokawa, T. Suda, Proc. Natl. Acad. Sci. USA 92, 6112 (1995); H. L. Henry, R. J. Midgett, A. W. Norman, J. Biol. Chem. 249, 7584 (1974)), and positively regulated by peptide hormones like calcitonin and PTH, which participate in calcium regulation (H. Kawashima, S. Torikai, K. Kurokawa, Nature 291, 327 (1981); K. W. Colston, L. M. Evans, L. Galauto, L. Macintyre, D. W. Moss, Biochem. J. 134, 817 (1973); D. R. Fraser and E. Kodicek, Nature 241, 163 (1973); M. J. Beckman, J. A. Johnson, J. P. Goff, T. A. Reinhardt, D. C. Beitz, R. L. Horst, Arch. Biochem. Biophys. 319, 535 (1995)). The complicated regulation of the 1α (OH)-ace activity by these hormones maintains the serum concentration of 1α,25(OH)₂D₃ at a certain level. The mutation of the 1α(OH)-ase gene may causes a genetic disease, vitamin D-dependent type I rickets (D. Fraser, S. W. Kooh, H. P. Kind, M. F. Hollick, Y. Tanaka, H. F. DeLuca, N. Engl. J. Med. 289, 817 (1973); S. Balsan, inRickets, F.M. Glorieux, Ed. (Raven, New York, 1991), pp155-165), which also demonstrate the importance of the enzyme *in vivo* in the function of vitamin D. The biochemical analysis of partially purified 1α(OH)-ase protein strongly suggested that this enzyme belongs to P450 family (S. Wakino et al., Gerontology 42,67 (1996); Eva Axen, FEBS Lett. 375, 277 (1995); M. BurgosTrinidad, R. Ismaii, R. A. Ettinger, J. M. Prahl, H. F. DeLuca, J. Biol. Chem. 267, 3498 (1992); M. Warner et at, J. Biol. Chem. 257, 12995 (1982)). However, the biochemical characteristics of the enzyme and the molecular mechanism of the negative feedback by 1α,25(OH)₂D₃ are not well understood since the enzyme purification is difficult and cDNA has not been cloned yet. Thus, the cDNA cloning of the enzyme had been desired. Recently, the cloning of the rat enzyme that hydroxylates the 1α position of vitamin D has been reported (J. Bone Min. Res. Vol. 11 (supl) 117 (1996)).

Conventionally, methods depend on phosphorylation of intracellular signal transduction factors or ion channels of membrane receptors as criteria have mainly been employed to screen genes encoding polypeptides that act on a specific nuclear receptor directly or indirectly, including 1α (OH)-ase mentioned above. Specifically, expression vectors into which a cDNA library or cDNA is inserted are introduced into cells or haploid individuals, for example Xenopus oocytes, and then phosphorylation, cell growth and the change in the electric current has been monitored for the screening.

However, it has been very difficult to isolate genes by using these methods. Especially, highly sophisticated techniques are required for the expression cloning of an enzyme itself because the indicators available for the detection are limited. Therefore, the development of a simple and efficient screening method has been desired.

### Disclosure of the Invention

An objective of the present invention is to provide a simple and efficient method for screening a gene encoding a polypeptide that converts a ligand precursor into a ligand, and a method for determining whether or not a test gene encodes a polypeptide that converts a ligand precursor into a ligand. Another objective of the present invention is to provide a method for isolating a polypeptide that converts a ligand precursor into a ligand and a gene encoding it, using the screening method.

Furthermore, an objective of the invention is to provide a method for screening a ligand that binds to a nuclear receptor, a method for determining whether or not a test compound is a ligand for a nuclear receptor, and a method for screening a gene encoding a polypeptide that converts an inactive form of a transcriptional regulatory factor into an active form, based on the screening method and the determination method described above.

The present inventors investigated to achieve the above objectives and focused on the nature of nuclear receptors, which function as transcriptional regulatory factor by being bound by a ligand. We successfully constructed the system in which a ligand is formed by the expression of a polypeptide that converts a ligand precursor into a ligand, and the ligand thus formed binds to a nuclear receptor to thereby induce the expression of a reporter gene located downstream of the target sequence. We searched a gene library using this system and succeeded in isolating a gene encoding a polypeptide capable of converting a ligand precursor into a ligand.

Specifically, the inventors constructed a vector comprising a gene encoding a fusion polypeptide of DNA binding domain of GAL4 and ligand-binding domain of vitamin D receptor and a vector in which the lacZ gene, a reporter, is located downstream of the binding sequence of the DNA binding domain of GAL4. These two vectors, and subsequently the cDNA library, were introduced into cells. Then the reporter activity was measured after adding the vitamin D precursor. Clones with the reporter activity were selected to examine whether or not they have the activities to convert the vitamin D precursor into vitamin D, thereby finding a clone that has the activity.

Also, the inventors found that this system, which takes the advantage of the transcriptional regulatory function of a nuclear receptor, makes it possible to screen a ligand that binds to a nuclear receptor and to examine whether or not a test compound is a ligand that binds to the nuclear receptor. Specifically, for example, a library of test compounds can be used in place of a ligand precursor and a gene library comprising the gene encoding a polypeptide that converts a precursor into a ligand in the system described above. When a test compound functions as a ligand, the nuclear receptor promotes the transcription of the reporter gene. Thus, compounds that function as ligands can be screened from the library simply by detecting the reporter activity as an index.

Furthermore, the inventors found that the system utilizing the transcriptional regulatory function of a nuclear receptor can be employed to screen genes that encode polypeptides capable of converting an inactive form of a wide range of transcriptional regulatory factors into an active form. In other words, the inventors found that the system in which the transcriptional regulatory function can be used to isolate factors involved in activation of various transcriptional regulatory factors, which have inactive and active forms, such as transcriptional regulatory factors activated by phosphorylation as well as nuclear receptors activated by the binding of ligands.

More specifically, this invention relates to:
1. a cell comprising a vector carrying a gene encoding a nuclear receptor and a vector carrying the binding sequence of the nuclear receptor and a reporter gene located downstream of said binding sequence;
2. the cell of 1, wherein the nuclear receptor is a vitamin D receptor;
3. a cell comprising a vector carrying a gene encoding a fusion polypeptide comprising DNA binding domain of a nuclear receptor and ligand-binding domain of a nuclear receptor, and a vector carrying the binding sequence of the DNA binding domain of the nuclear receptor and a reporter gene located downstream of the binding sequence;
4. the cell of 3, wherein the DNA binding domain of the nuclear receptor is originated from GAL4;
5. the cell of 3, wherein the ligand-binding domain of the nuclear receptor is originated from vitamin D receptor;
6. a method for screening a ligand that binds to a nuclear receptor, the method comprising
   (A) contacting a test compound with the cell of any one of 1 to 5,
   (B) detecting the reporter activity, and
   (C) selecting the test compound which elicited the reporter activity in the cell;
7. a method for determining whether or not a test compound is a ligand that binds to a nuclear receptor, the method comprising,
   (A) contacting a test compound with any one of the cell of 1 to 5, and
   (B) detecting the reporter activity;
8. a method for screening a gene encoding a polypeptide that converts a ligand precursor into a ligand, the method comprising
   (A) introducing a test gene into any one of the cell of 1 to 5,
   (B) contacting a ligand precursor to the cell into which the test gene is introduced,
   (C) detecting the reporter activity, and
   (D) isolating the test gene from the cell which showed the reporter activity;
9. a method for determining whether or not a test gene encoding a polypeptide that converts a ligand precursor into a ligand, the method comprising
   (A) introducing a test gene into any one of the cell of 1 to 5,
   (B) contacting a ligand precursor to the cell into which the test gene is introduced, and
   (C) detecting the reporter activity;
10. a method for screening a gene encoding a polypeptide that converts an inactive form of vitamin D₃ into an active form, the method comprising
   (A) introducing a test gene into the cell of 2 or 5,
   (B) contacting an inactive form of vitamin D₃ to the cell into which the test gene is introduced,
   (C) dectecting the reporter activity, and
   (D) isolating the test gene from the cell that shows the reporter activity;
11. a method for determining whether or not a test gene encodes a polypeptide that converts an inactive form of vitamin D₃ into an active form, the method comprising
   (A) introducing a test gene into the cell of 2 or 5,
   (B) contacting an inactive form of vitamin D₃ with the cell into which the test gene is introduced, and
   (C) detecting the reporter activity;
12. a ligand that binds to a nuclear receptor, which is obtainable by the method of claim 6;
13. a gene encoding a polypeptide that converts a ligand precursor into a ligand, which is obtainable by the method of claim 8.
14. a gene encoding a polypeptide that converts an inactive form of vitamin D₃ into an active form, which is obtainable by the method of claim 10.
15. a polypeptide comprising the amino acid sequence of SEQ ID NO: 1 or its derivative comprising said sequence in which one or more amino acids are substituted, deleted, or added, and having activity to convert an inactive form of vitamin D₃ into an active form;
16. a polypeptide comprising the amino acid sequence of SEQ ID NO: 2 or its derivative comprising said sequence in which one or more amino acids are substituted, deleted, or added, and having activity to convert an inactive form of vitamin D₃ into an active form;
17. a polypeptide encoded by a DNA that hybridizes with a DNA having the nucleotide sequence of SEQ ID NO: 3, wherein the polypeptide has activity to convert an inactive form of vitamin D₃ into an active form;
18. a polypeptide encoded by a DNA that hybridizes with the nucleotide sequence of SEQ ID NO: 4, wherein the polypeptide has activity to convert an inactive form of vitamin D₃ into an active form;
19. a DNA encoding any one of the polypeptide of 15 to 18;
20. a DNA hybridizing with a DNA having the nucleotide sequence of SEQ ID NO: 3 and encoding a polypeptide having activity to convert an inactive form of vitamin D₃ into an active form;
21. a DNA hybridizing with a DNA having the nucleotide sequence of SEQ ID NO: 4 and encoding a polypeptide having activity to convert an inactive form of vitamin D₃ into an active form;
22. a vector comprising any one of the DNA of 19 to 21;
23. a transformant expressively retaining any one of the DNA of 19 to 21;
24. a method for producing any one of the polypeptide of 15 to 18, the method comprising culturing the transformant of 23;
25. an antibody that binds to any one of the polypeptide of 15 to 18;
26. a method for screening a gene encoding a polypeptide that converts an inactive form of transcriptional regulatory factor into an active form, the method comprising
   (A) introducing a test gene into cells into which a vector comprising a gene encoding an inactive form of transcriptional regulatory factor and a vector comprising the binding sequence of said inactive transcriptional regulatory factor and a reporter gene located downstream thereof are introduced,
   (B) detecting the reporter activity, and
   (C) isolating the test gene from the cells showing the reporter activity;
27. a method of 26, wherein the inactive transcriptional regulatory factor is a complex of non-phosphorylated NFκB and IκB, non-phosphorylated HSTF, or non-phosphorylated AP1.

The term "ligand" used herein means a compound that binds to a nuclear receptor and regulates the transcriptional activating ability of a target gene of the nuclear receptor. It includes not only naturally-occurring compounds but also synthetic compounds.

The term "nuclear receptor" used herein means a factor that binds to an upstream site of a promoter of a target gene and ligand-dependently regulates transcription.

The "polypeptide that converts a ligand precursor into a ligand" includes a polypeptide that acts directly on a ligand precursor to convert it into a ligand. It also includes a polypeptide that indirectly converts a ligand precursor into a ligand, for example, a polypeptide activating a polypeptide that directly acts on a ligand precursor to convert it into a ligand.

The "transcriptional regulatory factor" used herein means a factor that binds to an upstream site of a promoter of a target gene and regulates transcription of the target gene. The above-described nuclear receptor is included in the transcriptional regulatory factor of the invention.

The "polypeptide that converts an inactive form of transcriptional regulatory factor into an active form" used herein includes not only a polypeptide that acts directly on an inactive form of transcriptional regulatory factor to convert it into an active form but also a polypeptide that indirectly converts an inactive form to an active form. When an inactive form of transcriptional regulatory factor is converted into an active form by phosphorylation, the transcriptional regulatory factor of the invention includes a polypeptide that activates a polypeptide phosphorylating the inactive form and indirectly converts the inactive form into the active form as well as a polypeptide directly involved in the phosphorylation.

The first aspect of the present invention relates to a method for screening a gene encoding a polypeptide that converts a ligand precursor into a ligand, and a method for determining whether or not a test gene encodes a polypeptide that converts a ligand precursor into a ligand. In these methods, a vector carrying a gene encoding a nuclear receptor (expression unit 1), and a vector carrying the binding sequence of the nuclear receptor and a reporter gene located downstream thereof (expression unit 2) are introduced into cells. Then, a test gene is introduced into the cells.

The "gene encoding a nuclear receptor" in the expression unit 1 is not particularly limited and any nuclear receptor gene can be used. For example, when orphan receptors such as PPAR, LXR, FXR, MB67, ONR, NUR, COUP, TR2, HNF4, ROR, Rev-erb, ERR, Ftz-F1, Tlx and GCNF (Tanpakusitsu Kakusan Koso (Protein, Nucleic Acid, Enzyme) Vol. 41 No. 8 p1265-1272 (1996)) are used as the nuclear receptor in the below-memtioned screening of unknown ligands that bind to nuclear receptors or determination whether or not a test compound is a ligand binding to a nuclear receptor, the naturally-occurring or synthesized ligand can be detected and isolated. Furthermore, nuclear receptors for which the ligand and ligand precursor are known, such as VDR (vitamin D receptor), ER, AR, GR, MR (Tanpakusitsu Kakusan Koso (Protein, Nucleic Acid, Enzyme) Vol. 41 No. 8 p1265-1272 (1996)) are preferably used in the below-mentioned screening of genes encoding polypeptides that convert a ligand precursor into a ligand or the determination whether or not a test gene encodes a polypeptide that converts a ligand precursor into a ligand. However, nuclear receptors used in the present invention are not limited thereto.

In the present invention, the nuclear receptor gene can be used alone, and a fusion polypeptide gene comprising the DNA binding domain of a nuclear receptor and the ligand-binding domain of another nuclear receptor can also be used. For example, the DNA binding domain of GAL4 is preferably used as the DNA binding domain because it enhances the expression of the reporter gene downstream thereof.

The "binding sequence of a nuclear receptor" in the expression unit 2 varies depending on the nuclear receptor. In most nuclear receptors, sequences comprising "AGGTCA" are usually used. In the case of a dimeric nuclear receptor, the binding sequence is preferably composed of two repetition of the sequence. The repetitive sequences include the direct-repeat type, in which the two sequences are aligned in the same direction, and the palindrome type, in which the sequences are directed to the center (Tanpakusitsu Kakusan Koso (Protein, Nucleic Acid, Enzyme) Vol. 41 No. 8 p1265-1272 (1996)). A spacer sequence usually exists between the repetition sequences, which can determine the specificity of the nuclear receptor (K. Umesono et al., Cell Vol. 65, p1255-1266 (1991)).

A reporter gene located downstream of a nuclear receptor is not particularly limited. Preferable reporter genes are, for example, lacZ, CAT, and luciferase. Resistant genes to toxins or antibiotics, such as ampicillin resistant gene, tetracycline resistant gene, kanamycin resistant gene, can also be used to select cells by applying the corresponding toxin or antibiotic.

The binding sequence of a nuclear receptor and the reporter gene are not necessarily connected directly. Some sequences that alter the strength of the promoter, for example, the promoter region of β-globin, can be inserted between the binding sequence and the reporter gene.

Animal cells are preferable for introducing these expression units. Cells with high transformation efficiency such as COS-1 cells and HeLa cells are particularly preferable. Vectors for animal cells such as "pcDNA3" (Invitrogen) are preferred to construct expression units. Vectors can be introduced into host cells by a known method such as calcium phosphate method, lipofection method, electroporation method and the like.

A test gene is introduced into cells thus prepared. A test gene is not particularly limited, and any genes whose capability of converting a ligand precursor into a ligand is detected can be used. Genes are screened from cells or cDNA libraries prepared from mRNA isolated from tissues or the like, which are expected to express an objective gene. For example, a gene encoding a polypeptide that converts vitamin D precursor into active vitamin D can be screened from a cDNA library derived from kidney or the like. In this case, a vector expressing adrenodoxin (ADX) and an vector expressing adrenodoxin reductase (ADR) are preferably introduced into cells together with a test gene so as to efficiently generate active vitamin D. A test gene can be inserted into an appropriate vector and introduced into cells. For example, preferable vectors are 'pcDNA3' (Invitrogen) mentioned above or the like.

Next, cells into which a test gene is introduced are contacted with a ligand precursor. As the ligand precursor, the one that acts on a nuclear receptor expressed by the expression unit 1 mentioned above is usually used. Examples of the ligand precursor include, without limitation, 25-hydroxyvitamin D₃, a precursor of VDR ligand (active vitamin D, 1α,25(OH)₂D₃); testosterone, a precursor of ER ligand (estrogen) and AR ligand (dihydroxytestosterone); 11-deoxycortisol, a precursor of GR ligand (cortisol), corticosterone, a precursor of MR ligand (aldosterone), etc. The contact of the ligand precursor with the cells can be performed by adding the ligand precursor to the culture medium of the cells, or a similar method.

The reporter activity is then detected. If a test gene that is introduced into cells encodes a polypeptide that converts a ligand precursor into a ligand, the ligand generates from the ligand precursor contacted with the cells, and binds to the nuclear receptor to make a ligand- nuclear receptor complex, which then binds to its target sequence to express the reporter gene. If the test gene does not encode a polypeptide that converts a ligand precursor into a ligand, the ligand is not produced from the ligand precursor and thus the reporter gene is not expressed. In this way, detecting the reporter activity enables judging whether or not the test gene encodes a polypeptide that converts a ligand precursor into a ligand. The reporter activity can be detected by a method well known in the art using criteria such as staining, fluorescence, or cell viability, depending on the reporter gene.

When a gene library or the like is used instead of a single gene, cells are selected by the reporter activity to isolate the test gene. The test gene can be extracted from cells by, for example, the method described in H. S. Tong et al., Journal of Bone and Mineral Research Vol. 9, 577-584 (1994). The primary structure of the gene extracted can be determined by a known method such as dideoxy method.

The cells into which expression units 1 and 2 are introduced can be used for screening genes encoding polypeptides capable of converting a ligand precursor into a ligand or determining whether or not a test gene encodes a polypeptide that converts a ligand precursor into a ligand. Furthermore, the cells can be used for screening ligands that bind to a nuclear receptor or determining whether or not a test compound is a ligand that binds to a nuclear receptor. Specifically, a candidate for a ligand that acts on a nuclear receptor (a single test compound or a library of test compounds) is used instead of a ligand precursor and a candidate for a gene encoding a polypeptide that converts the ligand precursor into the ligand (a single candidate gene, gene libraries, etc.). When a test compound functions as a ligand, a complex of a nuclear receptor and the test compound (ligand) activates the reporter located downstream of the target sequence and thus whether or not the test compound function as a ligand can be judged. Furthermore, compounds that function as ligands can be screened from plural compounds by detecting the reporter activity.

The inventors screened genes encoding polypeptides capable of converting the vitamin D precursor into active vitamin D as an example of the screening of genes encoding enzymes capable of converting a ligand precursor into a ligand, and obtained a desired gene. The present invention also relates to a polypeptide that converts the vitamin D precursor into active vitamin D and a gene encoding it.

Polypeptides derived from mouse and human that convert the vitamin D precursor into active vitamin D, which are encompassed by the polypeptides of the present invention, are shown in SEQ ID NO: 1 and SEQ ID NO: 2, respectively. Vitamin D is first hydroxylated in the liver to generate 25(OH)D₃, then hydroxylated in the kidney to generate 1α,25(OH)₂D₃. The polypeptide of the present invention converts 25(OH)D₃ into 1α,25(OH)₂D₃ by hydroxylation, namely hydroxylates the 1α position of vitamin D (1α(OH)-ase).

The polypeptide of the present invention can be a naturally-occurring protein. Alternatively, it can be prepared as a recombinant polypeptide by gene recombination techniques. Both are included in the polypeptide of the present invention. A naturally-occurring protein can be isolated by methods well known in the art, for example, from kidney cell extract by affinity chromatography using an antibody binding to the polypeptide of the present invention as described below. On the other hand, a recombinant protein can be prepared by culturing cells transformed with a DNA encoding the polypeptide of the present invention as described below.

In addition, those skilled in the art can prepare polypeptides with substantially the same biological activity as the polypeptide set forth in SEQ ID NO: 1 (or SEQ ID NO: 2) by substituting amino acid(s) of the polypeptide or the like known method. The mutation of amino acids can occur spontaneously. The polypeptide of the present invention also includes the mutants of the polypeptide set forth in SEQ ID NO: 1 (or SEQ ID NO: 2) whose amino acid(s) are modified by substitution, deletion or addition, and which possesses the activity to convert the inactive form of vitamin D₃ into the active form. The known method of modifying an amino acid sequence is, for example, the method described in the literature, "Shin Saiboukougaku Jikken Protocol, Ed. Department of Oncology, The Institute of Medical Science, The University of Tokyo, p241-248" Mutations can be introduced by using commercially available 'QuickChange Site-Directed Mutagenesis Kit' (Stratagene).

It is a routine for those skilled in the art to prepare probes based on the entire or the partial nucleotide sequence of SEQ ID NO: 3 encoding the mouse polypeptide or SEQ ID NO: 4 encoding the human polypeptide, isolate DNAs with high homology with the probes from other species, and obtain polypeptides having the activities substantially equivalent to those of the polypeptide of the present invention using a known method such as hybridization technique (K. Ebihara et al., Molecular and Cellular Biology, Vol. 9, 577-584 (1994)) or polymerase chain reaction technique (S. Kitanaka et al., Journal of Clinical Endocrinology and Metabolism, Vol. 82, 4054-4058 (1997)). Therefore, the polypeptides of the present invention include those encoded by DNAs that hybridize with the DNA having the nucleotide sequence of SEQ ID NO: 3 or SEQ ID NO: 4, and having the activity to convert an inactive form of vitamin D₃ into an active form. Animal species used for isolating DNAs hybridizing with the DNA having the nucleotide sequence of SEQ ID NO: 3 or SEQ ID NO: 4 include rat, monkey, etc. DNAs encoding polypeptides with biological activities substantially equivalent to those of the polypeptide set forth in SEQ ID NO: 1 or SEQ ID NO: 2 usually have high homology with the DNA set forth in SEQ ID NO: 3 or SEQ ID NO: 4. The "high homology" means sequence identity of 70% or more, preferably 80% or more, and more preferably 90% or more. The homology of sequences can be calculated, for example, by the method described in Proc. Natl. Acad. Sci. USA 80, 726 (1983). An example of a method to isolate DNAs with high homology is as follows. The nucleotide sequence of SEQ ID NO: 3 (or SEQ ID NO: 4, which encodes the human polypeptide) is radiolabeled with ³²P and used as a template to screen a cDNA library of a desired species. The hybridization conditions are usually 10% formamide, 5x SSPE, 1x Denhardt's solution, 1x salmon sperm DNA, preferably 25% formamide, 5x SSPE, 1x Denhardt's solution, 1x salmon sperm DNA, and more preferably 50% formamide, 5x SSPE, 1x Denhardt's solution, 1x salmon sperm DNA.

Another aspect of the present invention relates to a DNA encoding the polypeptide of the present invention described above. The DNA of the present invention can be cDNA, genomic DNA, or synthetic DNA. It can be used not only to isolate a polypeptide with activities substantially equivalent to those of the polypeptide of the present invention from other species, but also to produce the polypeptide of the present invention as a recombinant polypeptide. Specifically, the DNA encoding the polypeptide of the present invention, for example, the DNA set forth in SEQ ID NO: 3 or SEQ ID NO: 4, is inserted into an appropriate vector, which are introduced into appropriate cells. The transformant cells are cultured to express the polypeptide, and the recombinant polypeptide is purified from the culture.

The cells used to produce the recombinant polypeptide include, for example, Escherichia coli and mammalian cells. The vectors used for expressing the recombinant polypeptide in the cells vary depending on host cells. For example, pGEX (Pharmacia) and pET (Novagen) are suitably used for E. coli, and pcDNA3 (Invitrogen) is used suitably for animal cells. These vectors can be introduced into the host cells by heat-shock, for example. The recombinant polypeptide can easily be purified from the transformant by glutathione-Sepharose affinity chromatography when pGEX (Pharmacia) is used, and by nickel-agarose affinity chromatography when pET (Novagen) is used.

Those skilled in the art can readily raise antibodies that bind to the polypeptide of the invention using the polypeptide prepared as described above. The polyclonal antibodies of present invention can be prepared by a well known method. For example, the polypeptide is injected into a rabbit or the like and IG fraction is purified by ammonium sulfate precipitation. Monoclonal antibodies can be produced by preparing hybridoma from spleen cells of mice immunized with the polypeptide of the present invention and myeloma cells and culturing the hybridoma to secrete the monoclonal antibody in the culture medium, intraperitoneally injecting the antibody obtained into an animal to obtain a large quantity of the antibody.

The polypeptides, DNA, and antibodies of the present invention can be applied as follows. The polypeptides and DNA of the present invention can be used for therapy and/or diagnosis of patients with low 1α(OH)-ase activity, such as patients with defects in 1α(OH)-ase or renal failure. The present inventors have identified the mutation of the DNA of the present invention in vitamin D-dependent type I rickets case, specifically, P382S (mutation from CCT to TCT), R335P (mutation from CGG to CCG), G125E (mutation from GGA to GAA), R107H (mutation from CGC to CAC). The present invention is also applicable to treat these patients. The mutations in the patients can be identified by extracting DNA from peripheral leukocytes of a patient, amplifying the DNA by PCR using the primer in which each exon is set as intron, and determining the nucleotide sequence or the DNA by direct sequencing method. The DNA of the present invention can be used in gene therapy. In this case, the DNA of the invention is inserted into an appropriate vector, and the vector is introduced into the body *in vivo* or *ex vivo*, using retrovirus method, liposome method, or adenovirus method. The polypeptides of present invention can be used as an immobilized enzyme to produce active vitamin D derivatives, that is, hydroxylate 1α position of vitamin D or its derivatives without a hydroxyl group at 1α position. Furthermore, the antibodies of the present invention can be used for therapy of such as vitamin D excessiveness, granulomatous diseases, and lymphoma as well as purification of the polypeptides of present invention.

The inventors also enabled screening genes encoding a polypeptide capable of converting an inactive form of various transcriptional regulatory factors into an active form using the above-described screening system of ligands binding to nuclear receptors. Therefore, the present invention also relates to a method for screening a gene encoding a polypeptide that converts an inactive form of a transcriptional regulatory factor into an active form.

There are several reports on the mechanism of the conversion of a transcriptional regulatory factor into its active form. For example, NFκB, a tissue specific factor, is bound to a factor named IκB in the cytoplasm. When it is treated with TPA, IκB dissociates, and NFκB translocates into a nucleus. Considering the effect of TPA treatment, the phosphorylation by protein kinase C is probably involved in the conversion of NFκB into an active form. In the case of HSTF, its phosphorylation level is low before the heat-shock, and is high after the heat-shock. This indicates that the phosphorylation is involved in the conversion of HSTF into its active form. Phosphorylation is also considered to be involved in the conversion of AP1 into its active form.

GAL4 is an inactive form when GAL80 binds thereto before the induction by galactose. After the induction by galactose, the complex dissociates and GAL4 becomes an active form. hsp90 binds to a glucocorticoid receptor before the hormone induction. After the induction, the complex dissociate to form an active form of glucocorticoid receptor (Jikken Igaku (Experimental Medicine) Vol. 7, No.4 (1989)).

The "polypeptides that convert an inactive form of a transcriptional regulatory factor into an active form" used herein includes polypeptides functioning in activation of transcriptional regulatory factors by dissociation of inhibitory factors, or by its qualitative alteration, such as phosphorylation. The "inactive form of a transcriptional regulatory factor" include, for example, a complex of non-phosphorylated NFκB and IκB, non-phosphorylated HSTF, non-phosphorylated AP1, as described above, but is not limited thereto.

In this screening method, a gene encoding an inactive form of a transcriptional regulatory factor, instead of a nuclear receptor gene, is introduced into a vector to construct the "expression unit 1" described above, and a vector into which the binding sequence of the transcriptional regulatory factor and a reporter gene downstream thereof is constructed as the "expression unit 2." The expression units are introduced into cells, and a test gene is introduced into the cells. If the test gene introduced has activity to convert an inactive form of the transcriptional regulatory factor into an active form, the inactive transcriptional regulatory factor, which is the product of the expression unit 1, is converted into the active form, and then active transcriptional regulatory factor binds to its binding sequence in the expression unit 2 to induce expression of the reporter gene. In contrast, when the test gene introduced does not have activity to convert an inactive transcriptional regulatory factor into an active form, the reporter gene in the expression unit 2 will not be induced. Therefore, one can judge whether or not a test gene has activity to convert an inactive transcriptional regulatory factor into its active form using the present screening method by detecting the reporter activity. Then a gene library is used as a test gene, one can isolate a gene encoding a polypeptide with the activity to convert an inactive form of transcriptional regulatory factor into an active form from the library.

### Brief Description of the Drawings

Figure 1 schematically shows the expression cloning system mediated by VDR.
Figure 2 is a graph showing the serum concentration of 1α,25(OH)₂D₃ in 3- and 7-week-old VDR+/+, VDR+I- and VDR-/- mice.
Figure 3 is a micrograph of cells stained with X-gal. (b) presents COS-1 cells transformed with a expression cDNA library; (a), negative control; (c), positive control; and (d) stained cells with cDNA that was extracted from the positive cells in (b) and amplified by PCR.
Figure 4 shows the putative amino acid sequence of CYP1AD. The first methionine is assigned as position 1. Asterisk indicates the terminal codon. Putative mitochondria targeting signal is surrounded by square. Underline indicates sterol binding domain. Dotted underline indicates hem-binding domain.
Figure 5 shows homology of 'CYP1AD' to rat 25(OH)-ase (CYP27) and mouse 24(OH)-ase (CYP24). Amino acid sequence homologies in sterol binding domain and hem-binding domain are also indicated.
Figure 6 shows a photograph of 10% SDS-PAGE pattern of CYP1AD protein translated *in vitro*.
Figure 7 shows the result of CAT assay for detecting *in vivo* activity of CYP1AD. The bottom panel shows a representative CAT assay, and the top panel shows the relative CAT activity as average and SEM from three independent experiments.
Figure 8 shows the normal phase HPLC analysis of 25(OH)D₃ metabolites.
Figure 9 shows the reverse phase HPLC analysis of 25(OH)D₃ metabolites.
Figure 10 shows the northern blot analysis for analyzing tissue distribution of CYP1AD transcripts.
Figure 11 shows the northern blot analysis of 3- and 7-week-old, VDR+/+, VDR+/- and VDR-/- mice, with(+) or without(-) overdosage of 1α,25(OH)₂D₃ (50 ng/mouse).
Figure 12 shows the relative amount of the hydroxylase gene in 3- or 7-week-old, VDR+/+, VDR+/- and VDR-/- mice, with(+) or without(-) overdosage of 1α,25(OH)₂D₃ (50 ng/mouse).

### Best Mode for Implementing the Invention

The present invention is demonstrated with reference to examples below, but is not to be construed being limited thereto.

### Example 1

### Isolation of cDNA encoding an enzyme that hydroxylates 1α position of vitamin D

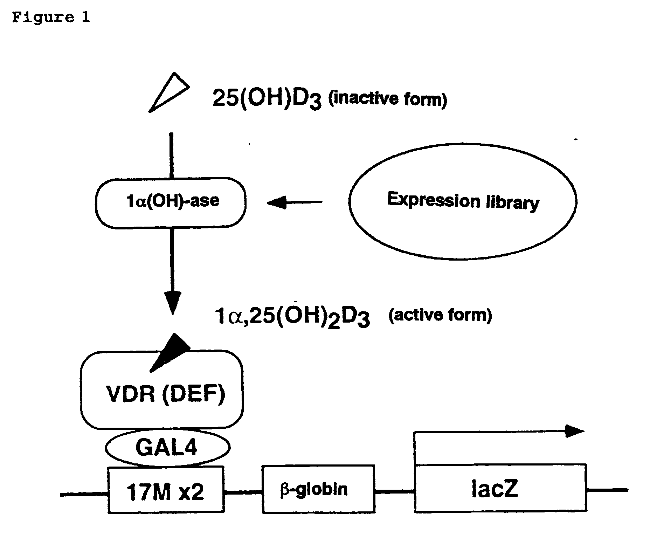

The inventors developed an expression cloning system mediated by a nuclear receptor for cloning a full-length cDNA encoding 1α (OH)-ase. The system is based on the mechanism that 25(OH)D₃, a precursor of 1α,25(OH)₂D₃, can activate the transactivating function of VDR only in the presence of 1α (OH)-ase (Figure 1). In other words, the ligand-dependent transactivating function of VDR (AF-2) is induced by 1α,25(OH)₂D₃, but not by 25(OH)D₃. 25(OH)D₃ is converted into 1α,25(OH)₂D₃ only in cells expressing 1α (OH)-ase. Therefore, the cells can be detected by X-gal staining (M. A. Frederick et al., Current Protocols in Molecular Biology (Wiley, New York, 1995)) as the result of the expression of the lacZ reporter gene in the presence of 25(OH)D₃.

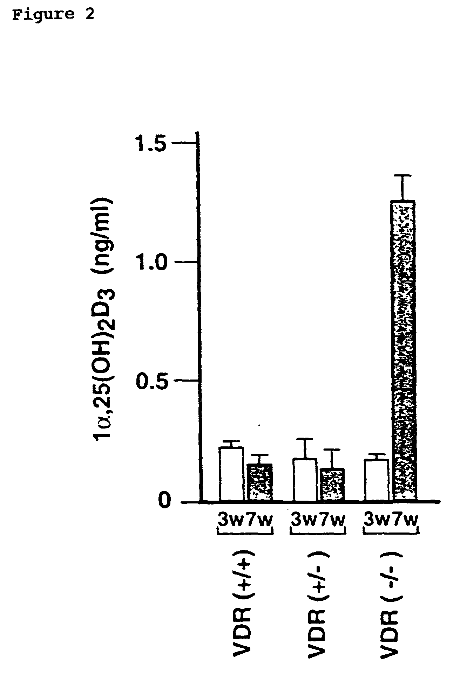

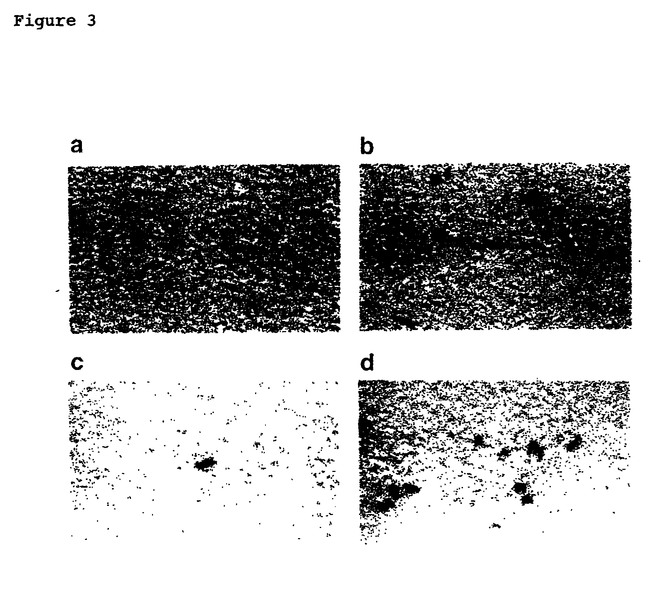

In the kidney of 7-week-old VDR-deficient mice (VDR-/- mice), the serum concentration of 1α,25(OH)₂D₃ was extremely high (Figure 2), which suggested the high 1α (OH)-ase activity. Therefore, the kidney of 7-week-old VDR-/- mice was used to prepare an expression library. poly(A)⁺ RNA was purified (K. Takeyama et al., Biochem. Biophys. Res. Commun. 222, 395 (1996); H. Mano et al., J. Biol. Chem. 269, 1591 (1994)), and total cDNA was prepared from poly(A)⁺ RNA (U. Gubler and B. J. Hoffman, Gene 25, 263 (1983); M. Kobori and H. Nojima, Nucleic Acid Res. 21, 2782 (1993)). The total cDNA was inserted into the HindIII position of pcDNA3 (Invitrogen), a expression vector that is derived from SV40, functions in mammals, and autonomously replicates in COS-1 cells. The reporter plasmid, 17M2-G-lacZ, was constructed by inserting yeast GAL4 (UAS) x2 and β-globulin promoter into the multicloning site of Basic expression vector (Clontech). The function of AF-2 induced by a ligand was detected using VDR-ligand-binding domain fused with GAL4-DNA binding domain (VDR-DEF) [GAL4-VDR(DEF)] (K. Ebihara et al., Mol. Cell. Biol. 16, 3393 (1996); T. Imai et al.., Biochem. Biophys. Res. Commun. 233, 765 (1997)). Cos-1 cells cultured in Dulbecco's Modified Eagle's Medium (DMEM) supplemented with 10% fetal calf serum were transiently transformed with 0.5 µg of GAL4-VDR (DEF) expression vector, 1 µg of 17M2-G.-lacZ, 0.2 µg each of ADX expression vector and ADR expression vector (T. Sakaki, S. Kominami, K. Hayashi, M. AkiyoshiShibata, Y. Yabusaki, J. Biol. Chem. 271, 26209 (1996); F. J. Dilworth et al., J. Biol. Chem. 270, 16766 (1995)), and 0.1 µg of the expression cDNA library, using Lipofectin (GIBCO BRL). 10⁻⁸M 25(OH)D₃ was added to the culture medium 12 hours after the transformation. Cells were fixed with 0.05% glutaraldehyde 48 hours after the transformation and were then incubated with X-gal at 37°C for 4 hours to identify β-galactosidase positive cells expressing 1α(OH)-ase by X-gal staining (Figure 3(c)) (M. A. Fredrick et al., Current Protocols in Molecular Biology (Wiley, New York, 1995)). In the negative control, the expression cDNA library was not used (Figure 3(a)). In the positive controls, the expression library was not used, and 1α,25(OH)₂D₃ was used instead of 25(OH)D₃ (Figure 3(b)).

The stained cells were selectively collected by micromanipulation using a micropipette with 40 µm diameter under an inverted microscope (H. S. Tong et al., J. Bone Miner. Res. 9, 577 (1994)), then transferred into PCR buffer solution. The PCR products were electrophoresed on 1% agarose gel, and fragments of about 2.0 to 2.5 kb, which is the expected cDNA size of the full-length 1α (OH)-ase, are purified and subcloned into pcDNA3. Sequence analysis of cDNA isolated from randomly selected 64 clones showed that 13 clones encode completely identical ORF. COS-1 cells into which the single cDNA clone was introduced were positive in X-gal staining (Figure 3(d)).

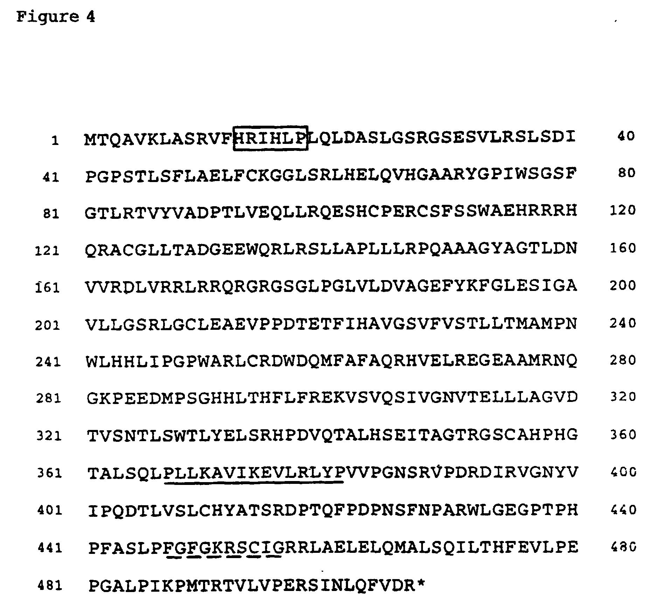

The full-length cDNA was obtained by the colony hybridization screening of the same library using the cDNA as a probe. The amino acid sequence deduced from ORF is a novel polypeptide with 507 amino acids (Figure 4).

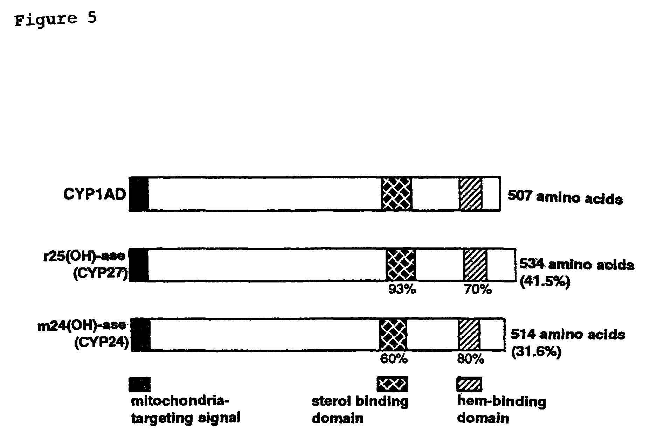

The polypeptide, hereinafter called "CYP1AD," has a mitochondria-targeting signal and has significant homologies with P450 family members (D. W. Nebert, DNA Cell. Biol. 10, 1 (1991)). Especially, the homology with rat vitamin D₃ 25-hydroxylase is 41.7% and that with mouse 25(OH)D₃ 24-hydroxylase is 31.6% (Figure 5)(O. Masumoto, Y. Ohyama, K. Okuda, J. Biol. Chem. 263, 14256 (1988); E. Usui, M. Noshiro, Y. Ohyama, K. Okuda, FEBS Lett. 262, 367 (1990); Y. Ohyama and K. Okuda, J. Biol. Chem. 266, 8690 (1991); S. Itoh et al., Biochem. Biophys. Acta. 1264, 26 (1995)). The homologies for sterol domain, especially conserved domain, in these enzymes are 93% and 60%, respectively, and those for hem binding domain are 70% and 80%, respectively.

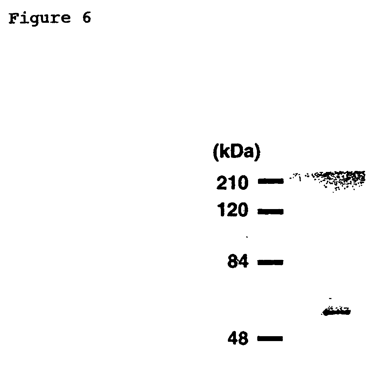

The 10% SDS-PAGE analysis of CYP1AD protein, which was translated *in vitro* in the presence of [³⁵S] methionine using Reticulocyte Lysate System (Promega) (H. Sasaki et al., Biochemistry 34, 370 (1995)) revealed that the molecular weight of the polypeptide is approximately 55 kDa (Figure 6), which is identical to the molecular weight of partially purified 1α (OH)-ase (S. Wakino et al., Gerontology 42, 67 (1996); Eva Axen, FEBS Lett. 375, 277 (1995); M. Burgos-Trinidad, R. Ismail, R. A. Ettinger, J. H. Prahl, H. F. DeLuca, J. Biol. Chem. 267, 3498 (1992); M. Warner et al., J. Biol. Chem. 257, 12995 (1982)).

### Example 2

### Detection of in vivo activity of CYP1AD

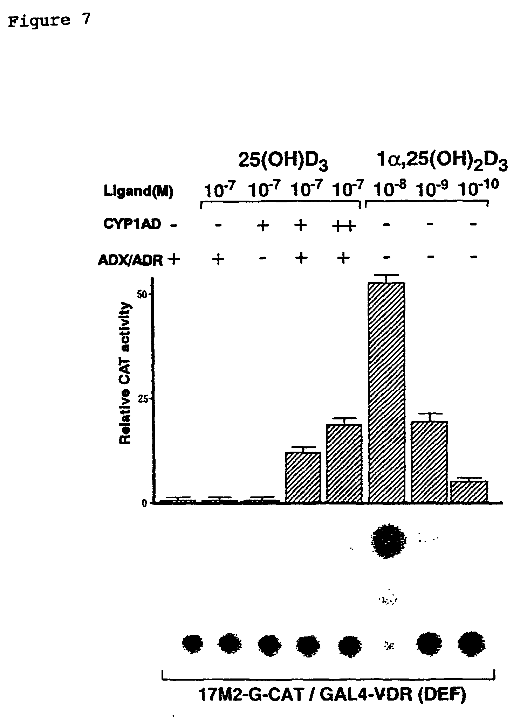

To confirm that CYP1AD has ability to activate the transactivating function of VDR by converting 25(OH)D₃ into active vitamin D *in vivo*, COS-1 cells were co-transformed with 0.5 µg of GAL4-VDR(DEF) expression vector, 1 µg of 17M2-G-CAT (S. Kato et al., Science 270, 1491 (1995)), 0.5 µg each of ADX expression vector and ADR expression vector (T. Sakaki, S. Kominami, K. Hayashi, H. Akiyoshi-Shibata, Y. Yabusaki, J. Biol. Chem. 271, 26209 (1996); F. J. Dilworth et al., J. Biol. Chem. 270, 16766 (1995)), and 1 µg of CYP1AD expression vector, in the presence of 25(OH)D₃ or 1α, 25(OH)₂D₃. A representative CAT assay is shown at the bottom panel of Figure 7. The relative CAT activities are shown at the top panel of Figure 7, as the average and SEM of three independent experiments. 25(OH)D₃ activated the CAT reporter gene when CYP1AD was expressed, while only 1α,25(OH)₂D₃ activated the reporter gene without using CYP1AD expression vector. However, 25(OH)D₃ did not significantly activate the reporter gene in the absence of ADX or ADR. These results strongly suggest that CYP1AD is 1α(OH)-ase, which converts 25(OH)D₃ into 1α,25(OH)₂D₃.

### Example 3

### Chemical analysis of CYP1AD products

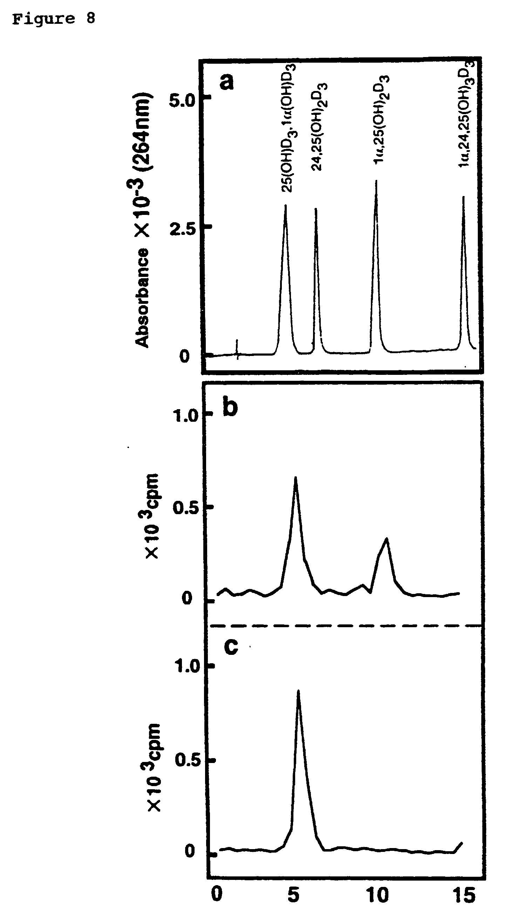

To chemically determine the enzyme product of CYP1AD, normal phase HPLC and reversed phase HPLC were performed (E. B. Mawer et al., J. Clin. Endocrinol. Metab. 79, 554 (1994); H. Fujii etal., EMBO J., in press (1997)). The cells (5x10⁶) transformed with ADR expression vector, ADX expression vector and CYP1AD expression vector (Figure 8(b)), or the cells (5x10⁶) not transformed (Figure 8(c)) were incubated in the presence of [³H]25(OH)D₃ (10⁵ dpm; 6.66 terabecquerel/mmol, Amersham International) at 37°C for 6 hours. The culture media were extracted with chloroform, and the extract was analyzed by normal phase HPLC using TSK-gel silica 150 column (4.6x250mm, Tosoh), with hexane/isopropanol/methanol (88:6:6) for mobile phase, at the flow rate of 1.0 ml/min. The eluate was collected and its radioactivity was measured using a liquid scintillation counter (E. B. Mawer et al., J. Clin. Endocrinol. Metab. 79, 554 (1994); H. Fujii et al., EMBO J. in press, (1997)). The standard samples of vitamin D derivatives, namely, 1α,(OH)D₃, 25(OH)D₃, 24,25(OH)₂D₃, 1α,25(OH)₂D₃ and 1α,24,25(OH)₃D₃, were applied to chromatography to determine their retention time by UV absorbance at 264 nm (Figure 8(a)).

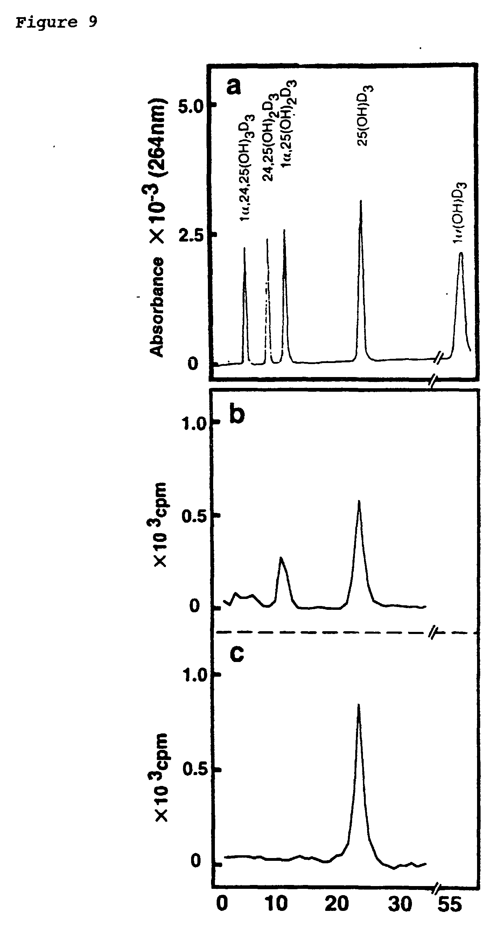

Likewise, reverse phase HPLC was performed with a column filled with Cosmasil 5C18-AR (4.6x150 mm Nacalai Tesque) at flow rate of 1.0 ml/min to confirm the existence of [³H]1α,25(OH)₂D₃. The chromatograms of standard samples for vitamin D derivatives, and the reaction product in the presence or absence of CYP1AD, are shown in Figure 9(a), (b), and (c), respectively.

The retention times of enzyme products in normal phase HPLC and reverse phase HPLC were completely identical to that of sample, 1α,25(OH)₂D₃ standard. The results indicate that the cDNA of CYP1AD encodes mouse 1α (OH)-ase, which hydroxylates 25(OH)D₃ to 1α,25(OH)₂D₃.

### Example 4

### Analysis of tissue distribution of CYP1AD transcripts

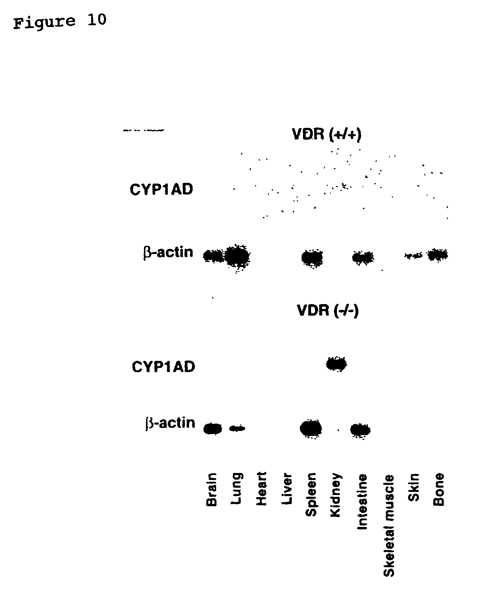

The tissue distribution of CYP1AD transcripts in 7-week-old normal and VDR-/- mice was examined. Poly(A)⁺ RNA was extracted from brain, lung, heart, liver, spleen, kidney, small intestine, skeletal muscle, skin, and bone, and analyzed by northern blot technique using cDNA of CYP1AD and β-actin as probes (K. Takeyama et al., Biochem. Biophys. Res. Commun. 222, 395 (1996); H. Mano et al., J. Biol. Chem. 269, 1591 (1994)). As the result, the transcript of CYP1AD was detected as a single band in the kidney. The size of the transcript (2.4 kbp) is identical to that of cloned cDNA (Figure 10). Except for kidney, in, 1α(OH)-ase activity has been reported in other tissues than kidney (A. W. Norman, J. Roth, L. Orchi, Endocr. Rev. 3, 331 (1982); H. F. DeLuca, Adv. Exp. Med. Biol. 196, 361 (1986); M. R. Walters, Endocr. Rev 13, 719 (1992); G. A. Howard, R. T. Turner, D. J. Sherrard, D. J. Baylink, J. Biol. Chem. 256, 7738 (1981); T. K. Gray, G. E. Lester, R. S. Lorenc, Science 204, 1311 (1979)). However, the transcript of 1α(OH)-ase was not detected in tissues other than kidney in this experiment.

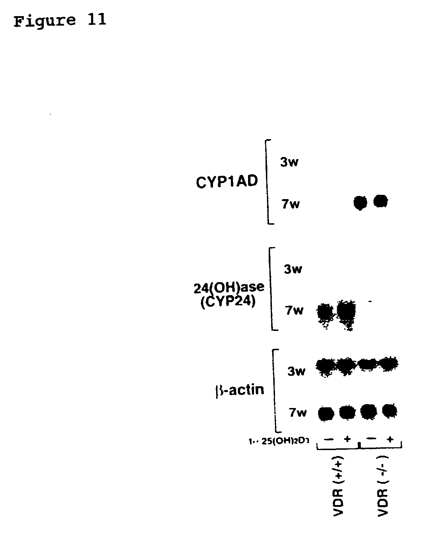

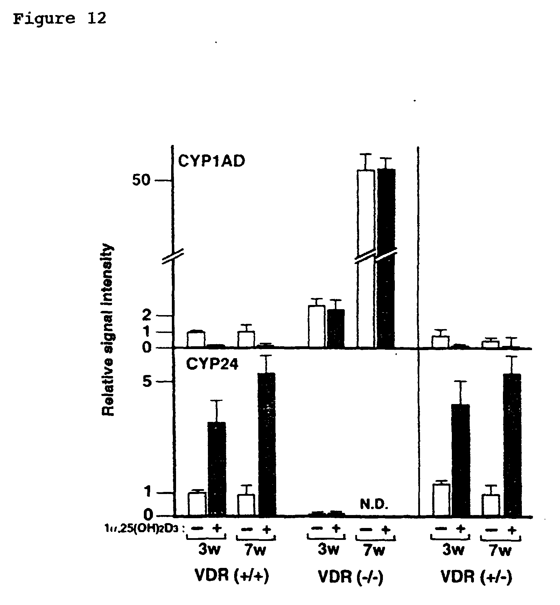

The northern blot analysis of the expression of the CYP1AD gene and the 24(OH)-ase (CYP24) gene was performed in 3- and 7-week-old VDR+/+, VDR+/-, and VDR-/- mice, with (+) or without (-) administration of excess 1α,25(OH)₂D₃ (50 ng/mouse). A representative northern blot analysis is shown in Figure 11. The relative amount of the hydroxylase gene standardized with the β-actin gene transcripts was measured in at least 5 mice for each group (Figure 12). Interestingly, the marked induction of the gene was seen in VDR-/- mice (2.5 and 50 times in 3- and 7-week-old mice, respectively)(Figure 11, 12). In VDR+/+ mice and VDR+/- mice, the administration of 1α,25(OH)₂D₃ significantly inhibited expression of the 1α (OH)-ase gene, whereas the inhibition did not occurred in 3- and 7-week-old VDR-/- mice. Therefore, the overexpression of 1α (OH)-ase appears to cause raise in the serum level of 1α,25(OH)₂D₃ in 7-week-old VDR-/- mice compared with the normal level (Figure 2). Considering these results, it can be considered that ligand-bound VDR is involved in the negative regulation of the 1α (OH)-ase gene expression by 1α,25(OH)₂D₃. In VDR-/- mice, the expression of the 24(OH)-ase gene was decreased to the undetectable level, and the reaction against 1α,25(OH)₂D₃ was not seen (Figure 11, 12). The 24(OH)-ase gene converts 25(OH)D₃ to 24,25(OH)₂D₃, which is an inactive form of vitamin D, and its gene expression is positively regulated by 1α,25(OH)₂D₃. These results confirmed that the ligand-bound VDR is involved in the gene expression induced by 1α,25(OH)₂D₃ through vitamin D responsive element in the promoter of the 24(OH)-ase gene (C. Zierold, H. M. Darwish, H. F. DeLuca, J. Biol. Chem. 270, 1675 (1995); Y. Ohyama et al., J. Biol. Chem. 269, 10545 (1994)). Therefore, the ligand-bound VDR adversely regulates the expression of 1α(OH)-ase and 24(OH)-ase genes by 1α,25(OH)₂D₃.

### Example 5

### Isolation of human gene encoding an enzyme that hydroxylates the 1α position of vitamin D

A normal human kidney cDNA library was prepared by extracting poly(A) RNA from normal human kidney tissue using the SacII(500bp)-Eco-RI(1200bp) fragment of mouse 1α (OH)-ase as a probe and inserting the RNA into λ-ZAPII. A human gene encoding the enzyme that hydroxylates 1α position of vitamin D was obtained by screening the library prepared above by plague hybridization method. The nucleotide sequence of the isolated gene is shown in SEQ ID NO: 4, and the putative amino acid sequence is shown in SEQ ID NO: 2.

### Industrial Applicability

The present invention provides a method for screening genes encoding polypeptides capable of converting a ligand precursor into a ligand, and a method for determining whether or not a test gene encodes a polypeptide that converts a ligand precursor into a ligand. The method of the present invention, unlike the existing expression cloning method, advantageously utilizes the nature of nuclear receptors that regulate transcription by being bound by a ligand. Since a desired gene can be detected by the reporter activity, the method of the invention enables simply and efficiently detecting and isolating a gene even if it encodes a polypeptide that is expressed at a low level. The present invention also provides a polypeptide that converts a ligand precursor into a ligand, namely, a polypeptide that converts an inactive form of vitamin D₃ into its active form and a gene encoding it, which are obtained by the screening method as described above. The polypeptide and gene of the present invention can be used for treating and/or preventing defects in 1α(OH)-ase or renal failure. The polypeptide of the present invention can also be used to produce active vitamin D derivatives, namely, hydroxylate 1α position of vitamin D or its derivatives without a hydroxyl group at 1α position. The antibodies against the polypeptide of the present invention can be used to purify the polypeptide of the present invention, and to treat vitamin D excessiveness, granulomatous diseases, lymphoma, and the like.

In addition, the present invention provides a method for screening ligands that bind to nuclear receptors, and a method for determining whether or not a test compound is a ligand of the nuclear receptor. The method also takes advantage of the nature of nuclear receptors and uses the reporter activity for the detection. These methods are thus simple and efficient as well as the method described above. For example, the method is useful in searching ligands for orphan- receptors, for which ligands are unknown.

Furthermore, the present invention provides a method for screening genes encoding polypeptides capable of converting an inactive form of transcriptional regulatory factor into an active form, based on the screening method described above. This method enables easily isolating genes that encode polypeptides capable of converting an inactive form of various transcriptional regulatory factors into the active form by detecting the reporter activity.

## Claims

1. A cell comprising a vector carrying a gene encoding a nuclear receptor and a vector carrying the binding sequence of the nuclear receptor and a reporter gene located downstream of said binding sequence.

2. The cell of claim 1, wherein the nuclear receptor is a vitamin D receptor.

3. A cell comprising a vector carrying a gene encoding a fusion polypeptide comprising DNA binding domain of a nuclear receptor and ligand-binding domain of a nuclear receptor, and a vector carrying the binding sequence of the DNA binding domain of the nuclear receptor and a reporter gene located downstream of the binding sequence.

4. The cell of claim 3, wherein the DNA binding domain of the nuclear receptor is originated from GAL4.

5. The cell of claim 3, wherein the ligand-binding domain of the nuclear receptor is originated from vitamin D receptor.

6. A method for screening a ligand that binds to a nuclear receptor, the method comprising
(A) contacting a test compound with the cell of any one of claims 1 to 5,
(B) detecting the reporter activity, and
(C) selecting the test compound which elicited the reporter activity in the cell.

7. A method for determining whether or not a test compound is a ligand that binds to a nuclear receptor, the method comprising,
(A) contacting a test compound with any one of the cell of claims 1 to 5, and
(B) detecting the reporter activity.

8. A method for screening a gene encoding a polypeptide that converts a ligand precursor into a ligand, the method comprising
(A) introducing a test gene into any one of the cell of claims 1 to 5,
(B) contacting a ligand precursor to the cell into which the test gene is introduced,
(C) detecting the reporter activity, and
(D) isolating the test gene from the cell which showed the reporter activity.

9. A method for determining whether or not a test gene encoding a polypeptide that converts a ligand precursor into a ligand, the method comprising
(A) introducing a test gene into any one of the cell of claims 1 to 5,
(B) contacting a ligand precursor to the cell into which the test gene is introduced, and
(C) detecting the reporter activity.

10. A method for screening a gene encoding a polypeptide that converts an inactive form of vitamin D₃ into an active form, the method comprising
(A) introducing a test gene into the cell of claims 2 or 5,
(B) contacting an inactive form of vitamin D₃ to the cell into which the test gene is introduced,
(C) dectecting the reporter activity, and
(D) isolating the test gene from the cell that shows the reporter activity.

11. A method for determining whether or not a test gene encodes a polypeptide that converts an inactive form of vitamin D₃ into an active form, the method comprising
(A) introducing a test gene into the cell of claims 2 or 5,
(B) contacting an inactive form of vitamin D₃ with the cell into which the test gene is introduced, and
(C) detecting the reporter activity.

12. A ligand that binds to a nuclear receptor, which is obtainable by the method of claim 6.

13. A gene encoding a polypeptide that converts a ligand precursor into a ligand, which is obtainable by the method of claim 8.

14. A gene encoding a polypeptide that converts an inactive form of vitamin D₃ into an active form, which is obtainable by the method of claim 10.

15. A polypeptide comprising the amino acid sequence of SEQ ID NO: 1 or its derivative comprising said sequence in which one or more amino acids are substituted, deleted, or added, and having activity to convert an inactive form of vitamin D₃ into an active form.

16. A polypeptide comprising the amino acid sequence of SEQ ID NO: 2 or its derivative comprising said sequence in which one or more amino acids are substituted, deleted, or added, and having activity to convert an inactive form of vitamin D₃ into an active form.

17. A polypeptide encoded by a DNA that hybridizes with a DNA having the nucleotide sequence of SEQ ID NO: 3, wherein the polypeptide has activity to convert an inactive form of vitamin D, into an active form.

18. A polypeptide encoded by a DNA that hybridizes with the nucleotide sequence of SEQ ID NO: 4, wherein the polypeptide has activity to convert an inactive form of vitamin D₃ into an active form.

19. A DNA encoding any one of the polypeptide of claims 15 to 18.

20. A DNA hybridizing with a DNA having the nucleotide sequence of SEQ ID NO: 3 and encoding a polypeptide having activity to convert an inactive form of vitamin D₃ into an active form.

21. A DNA hybridizing with a DNA having the nucleotide sequence of SEQ ID NO: 4 and encoding a polypeptide having activity to convert an inactive form of vitamin D₃ into an active form.

22. A vector comprising any one of the DNA of claims 19 to 21.

23. A transformant expressively retaining any one of the DNA of claims 19 to 21.

24. A method for producing any one of the polypeptide of claims 15 to 18, the method comprising culturing the transformant of 23.

25. An antibody that binds to any one of the polypeptide of claims 15 to 18.

26. A method for screening a gene encoding a polypeptide that converts an inactive form of transcriptional regulatory factor into an active form, the method comprising
(A) introducing a test gene into cells into which a vector comprising a gene encoding an inactive form of transcriptional regulatory factor and a vector comprising the binding sequence of said inactive transcriptional regulatory factor and a reporter gene located downstream thereof are introduced,
(B) detecting the reporter activity, and
(C) isolating the test gene from the cells showing the reporter activity.

27. The method of claim 26, wherein the inactive transcriptional regulatory factor is a complex of non-phosphorylated NFκB and IκB, non-phosphorylated HSTF, or non-phosphorylated AP1.
